# EUROPEAN PATENT SPECIFICATION

(11) **EP 1 227 076 B1**
(45) Date of publication and mention of the grant of the patent: **06.09.2006**
(21) Application number: 02001749.7
(22) Date of filing: 25.01.2002
(51) Int. Cl.: C07C 67/08, C07C 67/14, C07C 69/76, C07C 69/54, C09D 4/00, C07C 69/63, C07C 69/653, C07C 69/80

(54) **Hyperbranched fluorinated multifunctional alcohols and derivatives**
Hyperverzweigte fluorierte multifunktionale Alkohole und deren Derivate
Alcools fluorés hyperramifiés multifonctionels et leurs dérivés

(30) Priority: 25.01.2001 US 264200 P; 18.01.2002 US 50184 P
(43) Date of publication of application: 31.07.2002
(73) Proprietor: E.I. Du Pont de Nemours & Company, Wilmington, Delaware 19898 (US)
(72) Inventor: Wang, Fang, Tewksbury, MA 01876 (US); Xu, Chuck C., Tewksbury, MA 01876 (US); Xu, Baopei, 613, Wakefield, MA 01880 (US); Potterbaum, Indira, 17, Boston, MA 02215 (US)
(74) Representative: Hammond, Andrew David

(56) References cited:
- EP-A- 0 851 246
- EP-A- 1 229 352
- WO-A-00/78819
- WO-A-98/46556

## Description

### PRIORITY INFORMATION

This application claims priority from provisional application Ser. No. 60/264, 200 filed January 25, 2001.

### BACKGROUND OF THE INVENTION

The invention is directed to a class of hyperbranched fluorinated multifunctional alcohols and their derivatives such as acrylates and methacrylates, collectively referred to herein as acrylates, epoxies and vinyl ethers. The acrylates, epoxies and vinyl ethers are used in optical coatings and waveguide devices. Upon curing by thermal or photo initiation, these materials provide coatings of high crosslinking density, low surface tension, low birefringence, low refractive index, high optical clarity and high thermal stability.

Prior art references have disclosed that fluorine-containing polymers may be used for coating applications. See, for example, A.A. Wall, Fluoropolymers, Wiley-Interscience, 1972, and T. Deisenroth, Proc. Fluorine in Coatings II, Munich, 1997. The fluorinated polymers offer unique properties such as excellent chemical and thermal stability, good weathering and humidity resistance, low surface tension, low refractive index, and low absorption in the electromagnetic spectral region from 1300 to 1610 nm. The 1300-1610 nm region of the electromagnetic spectrum is particularly useful for fiber optic telecommunication networks. For example fluorinated photosensitive acrylates have been used to coat optical fibers as well as to fabricate optical waveguides.

It is well known in the art that actinic radiation such as UV light permits fast curing. UV curable compositions containing fluorinated monomers, oligomers and polymers have been widely reported. See, for example, US patents 4,508,916; 4,511,209; 4,914,171; 5,024,507; 5,062,680; 5,223,593; 5,822,489; European patent 333,464A1; and publications including J. Pacansky, Progress in Organic Coatings, 18 (1990) 79 and R. Bongiovanni, Progress in Organic Coatings, 36 (1999) 70. These compositions comprise fluorinated mono- or multi-functional acrylates and at least one photoinitiator.

Some fluorinated acrylate compositions in the prior art contain low molecular weight and mono-functional acrylates, however there are several drawbacks with these types of materials. First, the monomers do not have the minimum viscosity required to form a uniform coating having a certain thickness. Second, the high volatility of low molecular weight monomers impairs production of waveguides and other coatings. The high volatility monomer not only contaminates the curing chamber but also makes it difficult to achieve consistent material properties, including refractive index, after curing. Third, the low molecular weight of the monomers leads to very high shrinkage (up to 20%) upon curing. The high shrinkage causes high residual stress. Fourth, it is difficult to fully cure mono-functional monomers with UV light. The residual monomer will cause reliability and environmental problems.

Some fluorinated high molecular weight acrylates have been synthesized to overcome the above-mentioned problems. One example is the use of a urethane linkage to extend the molecular chain. However the chain-extended molecules have two acrylate groups per molecule. It is difficult to achieve fast curing and high cross-linking density when the acrylate groups are separated by a long molecular chain.

Therefore there is a need for an acrylate composition having high viscosity, low volatility, fast curing, high cross-linking density, low absorption in the wavelength region from 1300 to 1610 nm, low birefringence and low shrinkage upon curing.

### SUMMARY OF THE INVENTION

A fluorinated multifunctional alcohol synthesized from at least one core molecule having at least three equivalents of hydroxy-reacting functional groups and at least one fluorinated molecule having at least two hydroxyl groups. There are at least 1.5 equivalents of hydroxyl groups from the fluorinated molecule for every equivalent of hydroxy-reacting group from the core molecule.

One object of this invention is to provide fluorinated multifunctional alcohols synthesized from a core molecule having at least three equivalents of hydroxy-reacting functional groups and a fluorinated molecule containing at least two hydroxyl groups.

It is another object of the invention to provide a multifunctional alcohol which is a suitable precursor to fluorinated multifunctional acrylates, epoxies and vinyl ethers.

Another object of the invention is to provide new fluorinated multifunctional acrylates synthesized from the fluorinated multifunctional alcohols of the present invention.

It is still another object of the invention to provide an acrylate having high viscosity, low absorption from 1300 to 1600 nm, low volatility and low shrinkage upon curing.

### DETAILED DESCRIPTION OF PREFERRED EMBODIMENTS

Fluorinated multifunctional alcohols are synthesized from a core molecule having at least 3 equivalents of hydroxy-reacting functional groups and a fluorinated molecule containing at least 2 hydroxyl groups. There are at least 1.5 equivalents of hydroxyl groups for every equivalent of hydroxy-reacting group.

This is shown using the following non-exclusive reaction scheme: 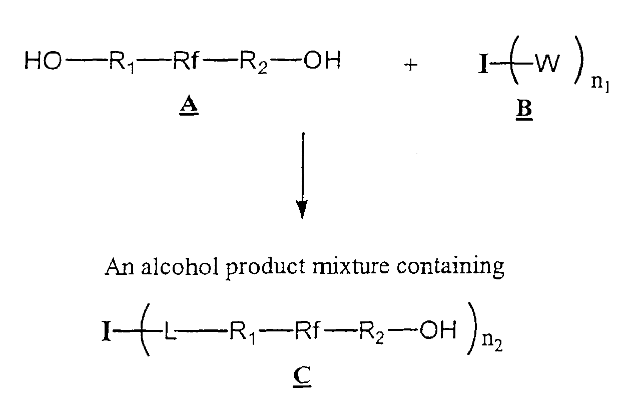 wherein **A** is a fluorinated monomer or polymer having 2 hydroxyl groups, Rf is a perfluorinated moiety; **B** is a multifunctional molecule having a core **I** wherein W stands for one equivalent of hydroxy-reacting group, and n₁ is at least 3; and **C** is the product mixture from **A** and **B,** wherein L is preferably an ether, ester or urethane link, and n₂ is at least 3.

When synthesizing **C** from **A** and **B,** the ratio of **A** to **B** is controlled so that there is a sufficient amount of alcohol **A** to prevent gelling and to form hydroxy terminated molecules. Typically there are 1.5-12.0, preferably 2.0-8.0 and more preferably 2.5-5,0 equivalents of OH groups from **A** for each equivalent of hydroxy-reacting group, **W**, from **B**. One equivalent of **W** herein means the amount of **W** required to consume each hydroxyl group. For example a carboxylic acid group has one equivalent of hydroxy-reacting group while an anhydride group has two equivalents of hydroxy-reacting groups. The molecular weight, molecular weight distribution and viscosity of the product can be tailored by changing the **A/B** ratio as will be understood by those skilled in the art.

The molecular structure given in the product **C** is the simplest form of the fluorinated multifunctional alcohols of the present invention. The product **C** is a mixture of alcohols. Their molecular weight distribution and molecular structure depend on the ratio between **A** and **B** as well as the reaction conditions. For example, some of the alcohol molecules produced contain multiple core units **I** due to di-, tri- or poly-condensation. The following generalized structures give examples of such products. These structures arise for three different values of n₁.

| | Mono-core | Di-core |
|---|---|---|
| n₁ = 3 | 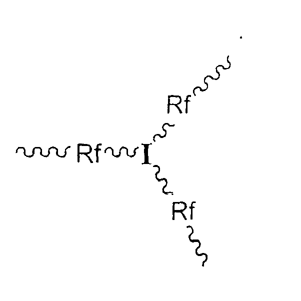 | 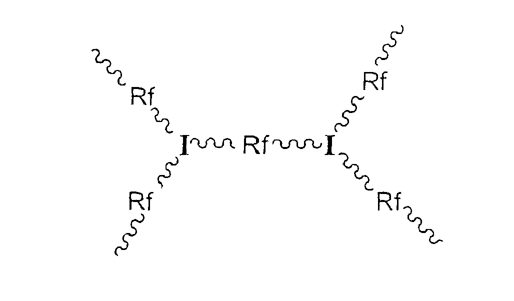 |
| n₁ =4 | 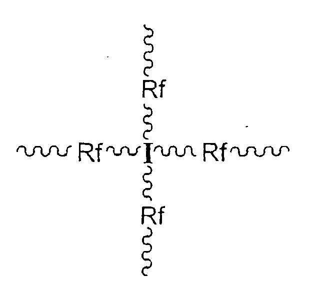 | 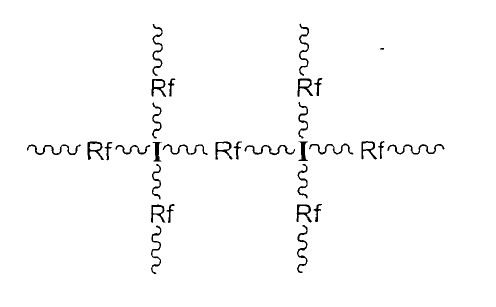 |
| n₁ =6 | 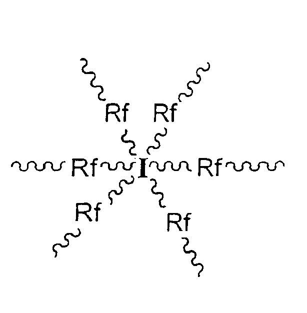 | 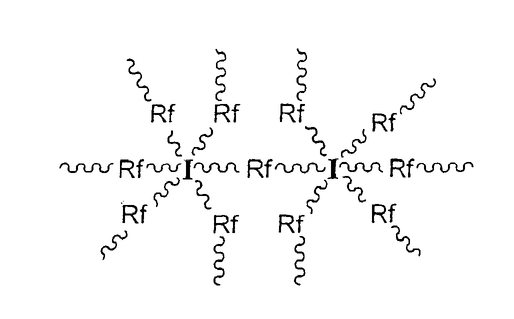 |

It is preferred that each free end of Rf is terminated with a hydroxyl group. The multifunctional alcohol product mixture **C** may also contain residual **A**. **A** can be either removed or retained depending on end uses.

**A** is a fluorinated diol, wherein Rf is a monomeric or polymeric perfluorinated alkylenediyl, oxyalkylene, arylene, oxyarylene, and mixtures thereof; R₁ and R₂ are monomeric or polymeric divalent moieties such as alkylenediyl, oxyalkylene, alkylene sulfide, arylene, oxyarylene, arylene sulfide, siloxane and mixtures thereof. Examples of suitable fluorinated diols include, but are not limited to, 1H, 1H, 9H, 9H-perfluoro-1,9-nonanediol, 1H, 2H, 3H, 3H-perfluorononane-1,2-diol, 1H, 1H, 10H, 10H-perfluoro-1,10-decanediol, 1H, 1H, 12H, 12H-perfluoro-1,12-dodecanediol, 1H, 1H, 16H, 16H-perfluoro-1,16-hexadecanediol, 1H, 1H, 8H, 8H-perfluorotetraethyleneglycol, fluoropoly(alkylene) diol, ethoxylated fluoropoly(alkylene) diols, fluoropoly(oxyalkylene) diols having the following structures:

HOCH₂CF₂O(CF₂CF₂O)ₙCF₂CH₂OH

HOCH₂CF₂CF₂O(CF₂CF₂CF₂O)ₙCF₂CF₂CH₂OH

HOCH₂CF₂CF₂CF₂O(CF₂CF₂CF₂CF₂O)ₙCF₂CF₂CF₂CH₂OH

HOCH₂CF₂O(CF₂CF₂O)ₘ(CF₂CF₂CF₂CF₂O)ₙ(CF₂CF₂O)ₘCF₂CH₂OH

and

HOCH₂CF₂O(CF₂CF₂O)ₘ(CF₂CF₂CF₂O)ₙCF₂CF₂O(CF₂CF₂CF₂O)ₙ(CF₂CF₂O)ₘCF₂CH₂OH

wherein m and n are integers, perfluoropolyether diols and ethoxylated perfluoropolyether diols such as Fluorolink D, D10, E and E10 commercially available from Ausimont USA and other variations known to those skilled in the art.

**B** is a multifunctional "core" molecule, wherein **I** is an aliphatic or aromatic moiety, **W** is a hydroxy-reacting functional group, such as a carboxylic acid and an alkyl halide, and n is at least 3 and preferably 3-6. When **B** reacts with **A** a new linkage L is formed. L can be either an ester, ether or urethane link depending on the type of the functional group **W**

To obtain an ester link **B** is chosen from multifunctional carboxylic compounds such as carboxylic acids, acid chlorides, anhydrides, esters and other compounds known to those skilled in the art. These compounds react with diols, **A,** to form polyesters **C** with hydroxy end groups. Suitable "core" compounds, **B**, include, but are not limited to, multifunctional acids such as 1,3,5-cyclohexanetricarboxylic acid, Kemp's triacid, 1,2,3-benzenetricarboxylic acid, 1,2,4-benzenetricarboxylic acid, 1,3,5-benzenetricarboxylic acid, 5-(4-carboxy-2-nitrophenoxy)-isophthalic acid, 1,2,3,4-butanetetracarboxylic acid, tetrahydrofuran-2,3,4,5-tetracarboxylic acid, 2,2',2",2"'-[1,2-ethanediylidene-tetrakis(thio)]-tetrakisacetic acid, cyclobutanetetracarboxylic acid, 1,2,4,5-benzenetetracarboxylic acid, mellitic acid, 1,4,5,8-naphthalene tetracarboxylic acid, and 1,2,3,4,5,6-cyclohexanehexacarboxylic acid; multifunctional esters such as methyl, ethyl or butyl esters of the above acids and triethylmethanetricarboxylate, triethyl 1,1,2-ethanetricarboxylate, tetraethyl 1,1,2,2-ethanetetracarboxylate, tetraethyl ethylenetetracarboxylate, tetramethyl exo,exo-tetracycloundeca-3,8-diene-3,4,8,9-tetracarboxylate, and pentamethyl cyclopentadiene-1,2,3,4,5-pentacarboxylate; anhydrides such as 1,2,4-benzenetricarboxylic anhydride, 1,2,4,5-benzenetetracarboxylic dianhydride, and bicyclo[2,2,2]oct-7-ene-2,3,5,6-tetracarboxylic anhydride; and acid chlorides such as 1,3,5-benzenetricarbonyl chloride.

To obtain an ether link, **B** is chosen from halides or other compounds that react with alcohols to form ether linkages such as those known to the skilled in the art. Non-exclusive examples of the halides include α,α,2,3,5,6-hexachloro-p-xylene, 1,3-dichloro-2-(chloromethyl)-2-methylpropane, 1,1,1-tris(chloromethyl)-propane, 2,4,6-tris(bromomethyl) mesitylene, pentaerythrityl tetrachloride, pentaerythrityl tetrabromide, 1,2,4,5-tetrakis(bromomethyl)-benzene and hexakis(bromomethyl)benzene.

The multifunctional acrylates are synthesized from the fluorinated multifunctional alcohols of the present invention. The following non-exclusive reaction illustrates the synthesis of the multifunctional acrylates. As discussed above, the alcohol product mixture of the present invention may contain one or more structures resulting from di-, tri- or poly-condensation. Such structures may contain 2, 3 or more core, **I**, units. Likewise, in addition to the structure shown in **E**, the acrylate mixture produced may contain one or more structures containing 2, 3 or more core, **I**, units. 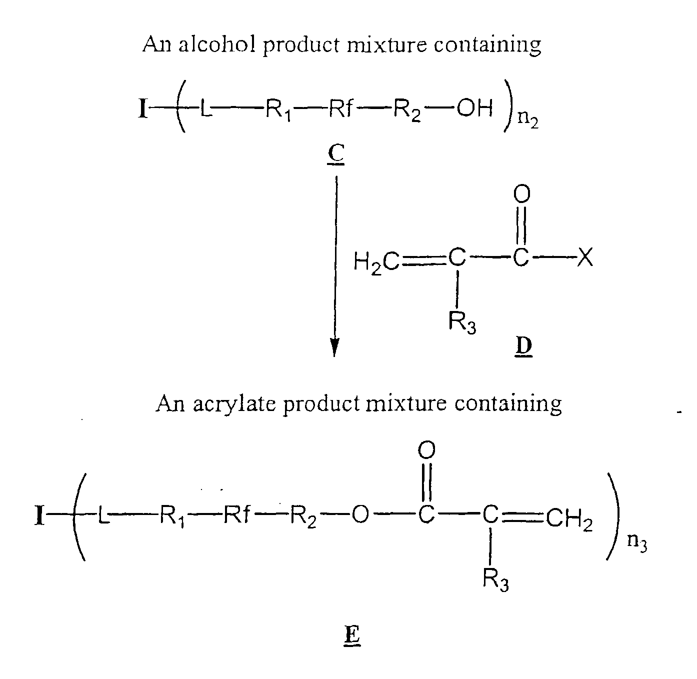 wherein **C** is the fluorinated multifunctional alcohol of the present invention described above, **D** is an acrylic acid, acrylic ester or acryloyl chloride and n₃ is at least 3. **C** and **D** react to form an acrylate mixture **E.** In one embodiment, the fluorinated alcohol **C** is converted into an acrylate **E** with acryloyl chloride using a tertiary amine. Preferably a hindered tertiary amine containing at least one tertiary or quaternary carbon atom is used. The hindered amine provides several advantages, as compared to commonly used triethylamine, such as reducing the yellowness of the products and eliminating the water washing process to remove ammonium salts formed during the acryloylation reaction. Non-exclusive examples of suitable hindered amines include N,N-dimethylisopropylamine, N,N-diisopropylethylamine, triisobutylamine, Julolidine, iminodibenzyl, 2-methylpyridine, 2,6-lutidine, 2,4,6-collidine, and mixtures thereof.

It is preferred that the fluorinated multifunctional acrylate has a number average molecular weight of at least 500 and more preferably at least 1000. The high molecular weight of the acrylate provides several advantages. First, the high molecular weight lowers shrinkage upon curing due to the relatively low volume fraction of the acrylate group. It is known in the art that low molecular weight acrylates can have shrinkage of up to 20% when cured leading to high residual stress. High residual stress causes problems such as birefringence, delamination, cracking, and light scattering. The acrylate composition of this invention has shrinkage of 5% or lower, preferably 2% or lower. Second, the high molecular weight corresponds with low volatility. Low volatility is important to reduce environmental and health risks and allows for a stable coating composition. Third, high molecular weight provides high viscosity. It is known in the art that a coating composition must have a minimum viscosity to yield high quality coatings with certain thickness.

In the acrylate discussed above, one end of the perfluorinated moiety, Rf, is pre-anchored to the core of the molecule. Such molecules allow for fast curing, high crosslinking density, and low shrinkage. Also, since n ≥ 3 in the formula of **E,** the acrylates produced are star shaped or hyper-branched. The star-shaped or hyper-branched molecules allow for isotropic films with low birefringence.

Although only the acrylate derivatives of the fluorinated multifunctional alcohol are described, other derivatives such as, but not limited to, epoxies and vinyl ethers can be synthesized from the same multifunctional alcohols.

A method of producing an optical coating comprises (1) forming a coating composition including at least one acrylate of this invention and at least one free radical initiator; (2) coating the composition into a film on a substrate having a substantially uniform thickness; and (3) curing the coating composition by exposure to an actinic radiation or heat, depending on the type of the initiator.

The coating composition is formed by thoroughly mixing the multifunctional acrylate of this invention with a free radical initiator and optionally other components such as, but not limited to, other acrylates and additives. The initiator can be either a photoinitiator, which generates free radicals when exposed to sufficient actinic radiation, or a thermal initiator, which generates free radicals when heated to a sufficient temperature. A composition containing a photoinitiator is herein called a photo-curable composition. A composition containing a thermal initiator is herein called a thermal curable composition.

The photo-curable composition contains at least one photoinitiator having a weight percentage of 0.1-12%, preferably 0.2-6.0% and more preferably 0.5-2.0%. The chosen photoinitiator is preferably thermally inactive below about 50°C. Examples of suitable photoinitiators include, but are not limited to, aromatic ketones, benzil ketals, benzoin, benzoin ethers, and phosphine oxides such as benzophenone, benzyl dimethyl ketal, benzoin alkylyl ethers, 1-hydroxy-cyclohexyl-phenyl ketone, benzodimethyl ketal, α,α-dimethyloxy-α-hydroxy acetophenone, 1-[4-(2-hydroxyethoxy)phenyl]-2-hydroxy-2-methyl-propan-1-one, 2-methyl-1-[4-methylthio)phenyl]-2-morpholino-propan-1-one, 2-benzyl-2-dimethylamino-1-(4-morpholinophenyl)-butan-1-one, and 2,4,6-trimethylbenzoyldiphenylphosphine oxide, and mixtures thereof. The preferred photoinitiator composition is a mixture of at least two photoinitiators with different extinction coefficients and absorption maxima. Such mixed photoinitiator composition enables high photo contrast as well as fast curing speed. Examples of such mixtures include, but are not limited to, benzodimethyl ketal with α,α-dimethyloxy-α-hydroxy acetophenone and 2,4,6-trimethylbenzoyldiphenylphosphine oxide with α,α-dimethyloxy-α-hydroxy acetophenone.

The thermal curable composition contains at least one thermal initiator at a weight percentage of 0.1-12%, preferably 0.2-6.0%, more preferably 0.5-2.0%. Suitable thermal polymerization initiators nonexclusively include peroxides such as benzoyl peroxide (BPO), di(sec-butyl)peroxydicarbonate, t-butyl peroxy-2-ethylhexanoate, t-butyl peroxyisobutyrate, 1,1-di-(amylperoxy)-cyclohexane, α-cumyl peroxyneodecanoate, t-amyl peroxyneodecanoate, laurolyl peroxide, dipropylperoxydicarbonate, decanoyl peroxide, cumene hydroperoxide, t-butyl cumyl peroxide, dicumyl peroxide, di-t-butyl peroxide, t-butyl hydroperoxide, di-t-butyl diperoxy-phthalate, t-amyl perbenzoate, t-butyl perbenzoate, t-butyl peroxyacetate, 2,5-dimethyl-2,5-di-(t-butylperoxy)hexane, 2,5-dihydroperoxy-2,5-dimethylhexane, t-amyl hydroperoxide, ethyl-3,3-di-(t-butylperoxy)-butyrate, 2,2-di-(t-butylperoxy)-butane and 2,2-di(t-amylperoxy)propane and other suitable thermal initiators include alkyl azo compounds wherein the alkyl group contains from 1 to 20 carbon atoms, such as 2,2-azobis-2-methylpropionitrile; and mixtures thereof.

Optional additives may be added to the thermo-curable or photo-curable composition of this invention to enhance certain properties such as thermal stability, chemical stability, coating quality, and photo contrast. Examples of the additives include, but are not limited to, surfactants, contrast enhancers, photo stabilizers, UV absorbers, antioxidants, and dyes. Examples of surfactants include fluorinated surfactants such as Fluorad from 3M of St. Paul, MN and polyethers such as BYK-3500 from BYK Chemie USA of Wallingford, CT. Suitable contrast enhancers include free radical scavengers particularly photo bleachable free radical scavengers such as the nitrones reported in US Patent 6,162,579. Photo stabilizers include hindered amines such as Cyasorb UV3346 from Cytec Industries of West Paterson, NJ and TINUVIN 123S from Ciba Specialty Chemicals of Tarrytown, NY. UV absorbers include benzotriazoles such as TINUVIN 234 from Ciba Specialty Chemicals and benzophenone derivatives such as UVINUL from BASF of Mount Olive, NJ. Antioxidants include for example hindered phenols such as Irganox 1010 from Ciba Specialty Chemicals. Dyes include methylene green, methylene blue and the like.

The resulting composition may be formed into thin films on a variety of substrates using methods well known in the art, including, but not limited to, spin coating, slot coating, dip coating and spray coating. Suitable substrates include, but are not limited to, silicon, glass, quartz, plastic, and metal. The polymer coating demonstrates high cross-linking density, high optical clarity, low birefringence, good thermal stability, low glass transition temperature and good adhesion to the substrates.

The photo curable composition is cured with an actinic radiation. The actinic radiation used can be any light in the visible and ultraviolet regions of the spectrum as well as electron beam. Preferably the actinic radiation is UV light. The UV sources, wavelengths, intensity, and exposure procedures may be varied to achieve the desired curing degree. Useful UV sources are high pressure xenon or mercury-xenon arc lamps fitted with appropriate optical filters. The thermal curable composition is cured by heating the coating to a sufficiently high temperature to generate free radicals from the thermal initiators.

### EXAMPLES

The following non-limiting examples are given only for the purpose of illustrating this invention. Variations in composition and synthetic methods will be apparent to those skilled in the art and are considered within the scope of this invention.

The following scheme depicts a specific method for the synthesis of a multifunctional fluorinated alcohol **F** and its acrylate **G** as described in Examples 1 and 2, respectively.

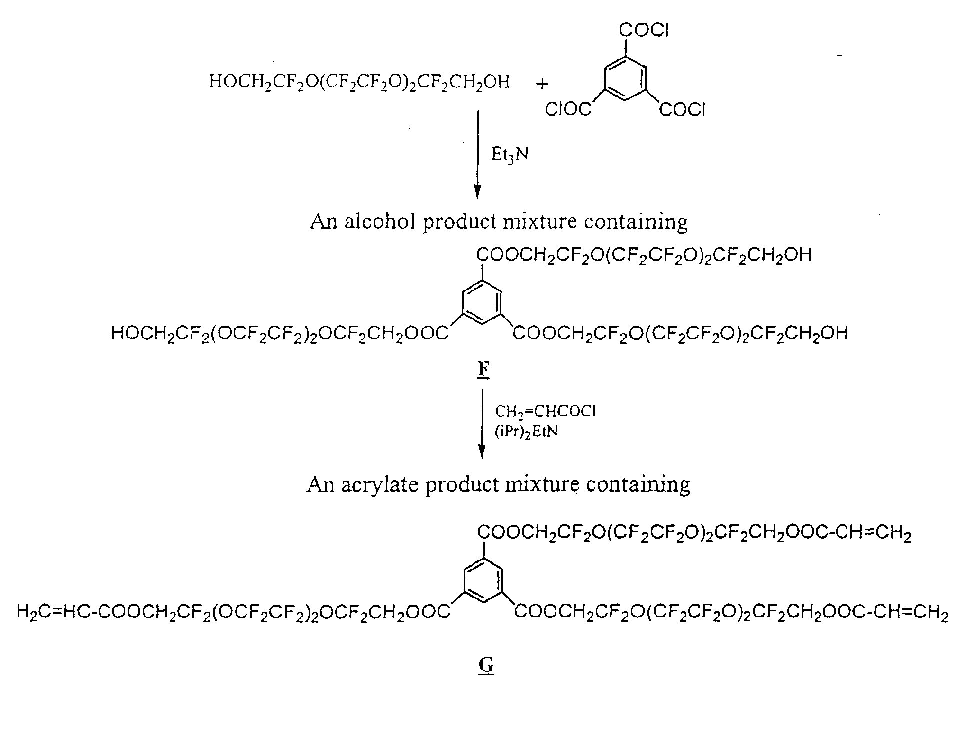

The molecular structures in the alcohol mixture, **F**, and the acrylate mixture, **G**, are for illustration only and only represent the structures of one of the molecules in the product mixtures. For example, some alcohols in F can contain more than one 1,3,5-benzenetricarbonyl core and more than three fluorinated tetraethylene oxide segments (as shown in the following structure). 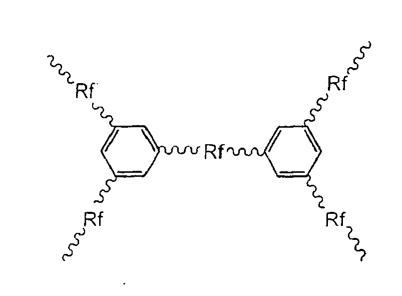 wherein Rf is -CF₂O(CF₂CF₂O)₂CF₂-.

**Example 1.** One equivalent of 1,3,5-benzenetricarbonyl trichloride and 4.5 equivalents of 1H, 1H, 8H, 8H-perfluorotetraethyleneglycol were dissolved in anhydrous ether (0.2 M 1,3,5-benzenetricarbonyl trichloride solution) in a cooled, argon-inerted three-neck flask while stirring. The flask was cooled with an ice-water bath. 3 equivalents of anhydrous triethylamine were dropwise added to this reaction mixture. After the addition of the triethylamine, the mixture was stirred for 2 hours at room temperature. The mixture was filtered through Celite to remove salt. The filtrate was concentrated to yield a fluorinated liquid alcohol.

**Example 2.** The alcohol prepared in Example 1 and 200 ppm hydroquinone were mixed in anhydrous t-butylmethylether (at 0.5 M hydroxyl group) in an argon-inerted three neck flask. To this reaction mixture, 1.2 equivalents of acryloyl chloride for each equivalent of hydroxyl group were added. Then 1.15 equivalents of anhydrous diisopropylethylamine was added dropwise while stirring. The reaction mixture was stirred for 10 h at room temperature. The reaction mixture was then quenched with 0.3 equivalent of methanol and stirred for 2 h to neutralize excess acryloyl chloride. The mixture was filtered through Celite/silica to remove salt. The filtrate solution was concentrated and pumped under vacuum at 60°C to yield a liquid acrylate. The crude product was then treated with 2% activated carbon to remove a light yellow color.

**Example 3**. One equivalent of 1,3,5-benzenetricarbonyl trichloride and 4.5 equivalents of Fluorolink D10, a fluorinated polyether diol commercially available from Ausimont USA, were dissolved in anhydrous t-butylmethylether (0.08 M 1,3,5-benzenetricarbonyl trichloride) in an argon-inerted three-neck flask while stirring in an ice-water bath. 3 equivalents of anhydrous diisopropylethylamine were added dropwise to the reaction mixture. After addition of the diisopropylethylamine, the mixture was stirred for 2 h at room temperature. Then 7.2 equivalents of acryloyl chloride were added to the stirred mixture, followed by 6.9 equivalents of anhydrous diisopropylethylamine. The reaction mixture was stirred for an additional 6h at 50°C. Finally the reaction mixture was quenched with 3.6 equivalents of methanol and stirred for 2 h to neutralize excess acryloyl chloride. The mixture was filtered through Celite/silica to remove salt. The filtrate solution was concentrated and pumped under vacuum at 60°C to yield a liquid acrylate. The crude product was then treated with 2% activated carbon to remove a light yellow color.

**Reference Example 4.** One equivalent of isophthaloyl dichloride and 2 equivalents of fluorinated tetraethylene glycol, available from Exfluor-Research, were dissolved in anhydrous ether (0.2 M isophthaloyl dichloride) in a cooled, argon-inerted three-neck flask while stirring. The flask was cooled with an ice-water bath. 3 equivalents of anhydrous triethylamine were dropwise added to the reaction mixture. After the addition of the triethylamine, the mixture was stirred for 2 h at room temperature. The mixture was then filtered through Celite to remove salt. The filtrate was concentrated to yield a viscous fluorinated liquid alcohol.

**Reference Example 5.** The fluorinated alcohol prepared in Example 4 and 200 ppm hydroquinone were mixed with anhydrous t-butyl methyl ether (at 0.5 M hydroxyl group) in an argon-inerted three neck flask. To this reaction mixture were added 1.2 equivalents of acryloyl chloride for each equivalent of hydroxyl group. Then 1.15 equivalents of anhydrous diisopropylethylamine were added dropwise while stirring. The reaction mixture was stirred for an additional 10 h at room temperature. The reaction mixture was then quenched with 0.3 equivalent of methanol and stirred for 2 h to neutralize excess acryloyl chloride. Finally the mixture was filtered through Celite/silica to remove salt, and the filtrate solution was concentrated and pumped under vacuum at 60°C to yield a fluorinated liquid acrylate. The crude product was treated with 2% activated carbon to remove a light yellow color.

**Example 6**. One equivalent of 1,3,5-benzenetricarbonyl trichloride and 4.5 equivalents of Fluorolink D10 were dissolved in anhydrous ether (0.2 M 1,3,5-benzenetricarbonyl Trichloride) in an argon-inerted three-neck flask while stirring in an ice-water bath. To this reaction mixture was added dropwise 3 equivalents of anhydrous triethylamine. After the addition of the triethylamine the mixture was stirred for 2 h at room temperature. Then the mixture was filtered through Celite to remove salt. The filtrate was concentrated to yield a viscous fluorinated liquid alcohol.

**Example 7.** One equivalent of trimethyl-1,3,5-benzenetricarboxylate, 0.06 equivalent sodium methoxide and 4.5 equivalents of Fluorolink D10 were mixed in an argon-inerted three-neck flask while stirring. The reaction mixture was heated to 115°C for about 20 h. Reduced pressure (100 mmHg) was used to remove the methanol generated during the reaction. Concentrated HC1 in ethyl acetate was added to neutralize excess base. The solution was dried over MgSO₄, filtered and concentrated to yield a viscous fluorinated liquid alcohol with a slight yellow color.

**Example 8.** The alcohol prepared in Examples 6-7 and 200 ppm hydroquinone were mixed with anhydrous t-butyl methyl ether (at 0.5 M hydroxyl group) in an argon-inerted three neck flask. To this reaction mixture were added 1.2 equivalents of acryloyl chloride for each equivalent of hydroxyl group. Then 1.15 equivalents of anhydrous diisopropylethylamine were added dropwise while stirring. The reaction mixture was stirred for 10 h at room temperature. The reaction mixture was quenched with 0.3 equivalent of methanol, stirred for 2 h to neutralize excess acryloyl chloride, and filtered through Celite/silica to remove salt. The filtrate solution was concentrated and pumped under vacuum at 60°C to yield a liquid acrylate. The crude product was then treated with 2% activated carbon to remove a light yellow color.

**Example 9.** One equivalent of 1,2,4-benzenetricarboxylic acid, Amberlyst-15 ion-exchange resin (40wt% of the 1,2,4-benzenetricarboxylic acid) and 4.5 equivalents of Fluorolink D10 were mixed in a three-neck flask, which was fitted with a water collecting apparatus, a nitrogen gas inlet and a gas outlet. The reaction mixture was heated to 200°C until the expected amount of water had been collected. The mixture was then cooled to room temperature and filtered through Celite. A viscous fluorinated liquid alcohol was obtained.

**Example 10.** The alcohol prepared in Example 9 and 200 ppm hydroquinone were mixed with anhydrous t-butyl methyl ether (at 0.5 M hydroxyl group) in an argon-inerted three neck flask. To this reaction mixture was added 1.2 equivalents of acryloyl chloride for each equivalent of hydroxyl group. 1.15 equivalents of anhydrous diisopropylethylamine were added dropwise while stirring. The reaction mixture was stirred for 10 h at room temperature. Finally the reaction mixture was quenched with 0.3 equivalent of methanol, stirred for 2 h to neutralize excess acryloyl chloride, and filtered through Celite/silica to remove salt. The filtrate solution was concentrated and pumped under vacuum at 60°C to yield a liquid acrylate. The crude product was then treated with 2% activated carbon to remove a light yellow color.

**Example 11.** One equivalent of tetraethyl-1,1,2,2-ethanetetracarboxylate, 0.08 equivalent of sodium ethoxide and 4.5 equivalents of Fluorolink D10 were mixed in an argon-inerted three-neck flask with a magnetic stirring bar. The reaction mixture was heated to 100°C for 2 h to remove the ethanol generated. Vacuum (50 mmHg) was then applied for 4 h to drive the reaction to completion. Concentrated HCl in ethyl acetate was added to neutralize excess base. The solution was dried over MgSO₄, filtered and concentrated to yield a viscous fluorinated liquid alcohol with a slight yellow color.

**Example 12**. The alcohol prepared in Example 11 and 200 ppm hydroquinone were mixed with anhydrous t-butyl methyl ether (at a 0.5 M of hydroxyl group) in an argon-inerted three neck flask. 1.11 equivalents of acryloyl chloride for each equivalent of hydroxyl group was added to the reaction mixture. 1.1 equivalents of anhydrous diisopropylethylamine were added dropwise while stirring. This reaction mixture was stirred for 10 h at room temperature. The reaction mixture was quenched with 0.4 equivalent of methanol, stirred for 2 h to neutralize excess acryloyl chloride, and filtered through Celite/silica to remove salt. The filtrate solution was concentrated and pumped under vacuum at 60°C to yield a liquid acrylate. The crude product was then treated with 2% activated carbon to remove a light yellow color.

**Example 13.** One equivalent of 1,2,3,4-butanetetracarboxylic acid, Amberlyst-15 ion-exchange resin (40 wt% of the 1,2,3,4-butanetetracarboxylic acid) and 6 equivalents of Fluorolink D10 were mixed in a three-neck flask, which was fitted with a water collecting apparatus. The reaction mixture was heated to 210°C until the expected amount of water had been collected. The mixture was cooled to room temperature and filtered through Celite to yield a viscous fluorinated liquid alcohol with a slight yellow color.

**Example 14.** The alcohol prepared in Example 13 and 200 ppm hydroquinone was mixed with anhydrous t-butyl methyl ether (at a 0.5 M of hydroxyl group) in an argon-inerted three neck flask. 1.11 equivalents of acryloyl chloride for each equivalent of hydroxyl group was added to the reaction mixture. 1.1 equivalents of anhydrous diisopropylethylamine were added dropwise while stirring. This reaction mixture was stirred for 10 h at room temperature. The reaction mixture was quenched with 0.4 equivalent of methanol, stirred for 2h to neutralize excess acryloyl chloride, and filtered through Celite/silica to remove salt. The filtrate solution was concentrated and pumped under vacuum at 60°C to yield a liquid acrylate. The crude product was then treated with 2% activated carbon to remove a light yellow color.

**Example 15.** One equivalent of 1,2,4,5-benzenetetracarboxylic dianhydride, Amberlyst-15 ion-exchange resin (40wt% of the 1,2,4,5-benzenetetracarboxylic dianhydride) and 6 equivalents of Fluorolink D10 were mixed in a three-neck flask, which was fitted with a water collecting apparatus. The reaction mixture was heated to 230°C until the expected amount of water had been collected. The mixture was cooled to room temperature and filtered through Celite to yield a viscous fluorinated liquid alcohol with.a slight yellow color.

**Example 16.** The alcohol prepared in Example 15 and 200 ppm hydroquinone was mixed with anhydrous t-butyl methyl ether (at a 0.5 M of hydroxyl group) in an argon-inerted three neck flask. To the reaction mixture were added 1.11 equivalents of acryloyl chloride for each equivalent of hydroxyl group. 1.1 equivalents of anhydrous diisopropylethylamine were added dropwise while stirring. This reaction mixture was stirred for 10 h at room temperature. The reaction mixture was quenched with 0.4 equivalent of methanol, stirred for 2 h to neutralize excess acryloyl chloride, and filtered through Celite/silica to remove salt. The filtrate solution was concentrated and pumped under vacuum at 60°C to yield a liquid acrylate. The crude product was treated with 2% activated carbon to remove a light yellow color.

**Example 17**. The acrylate of Example 2 was mixed with 0.5% Darocur 4265, a free radical photoinitiator from Ciba Specialty Chemicals, to form a photosensitive composition. The photosensitive composition was coated on a glass substrate and exposed to 500 mJ UV at 365 nm using a 1000W mercury xenon lamp to form a thin polymer coating. The coating was clear and colorless.

**Example 18**. The acrylate of Example 2 was mixed with 0.5% benzoyl peroxide (BPO), a free radical initiator, to form a thermo-curable composition. The composition was coated on a silicon substrate and heated at 90°C for 2 h to form a thin solid film. The coating was clear and colorless.

Although the present invention has been shown and described with respect to several preferred embodiments thereof, various changes, omissions and additions to the form and detail thereof, may be made therein, without departing from the scope of the invention as defined by the appended claims.

## Claims

1. A fluorinated multifunctional alcohol synthesized from at least one core molecule having at least three equivalents of hydroxy-reacting functional groups and at least one fluorinated molecule having at least two hydroxyl groups.

2. The multifunctional alcohol of Claim 1 wherein there are at least 1.5 equivalents of hydroxyl groups from the-fluorinated molecule for every hydroxy-reacting group from the core molecule.

3. The multifunctional alcohol of Claim 1 synthesized using the reaction scheme: 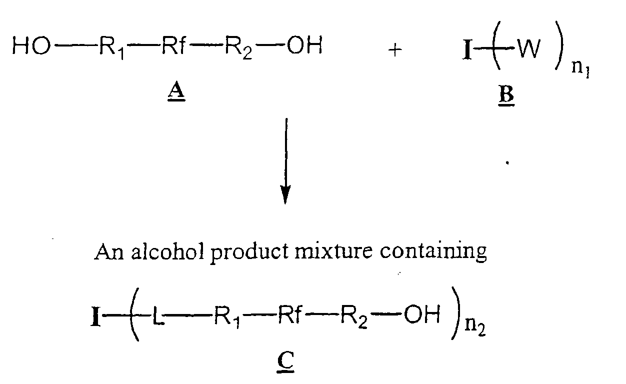 wherein **A** is a fluorinated monomer or polymer having two hydroxyl groups, wherein Rf is a monomeric or polymeric perfluorinated alkylenediyl, oxyalkylene, arylenediyl, oxyarylene, and mixtures thereof, and R₁ and R₂ are monomeric or polymeric divalent moieties such as alkylenediyl, oxyalkylene, alkylene sulfide, arylenediyl, oxyarylene, arylene sulfide, siloxane, and mixtures thereof; **B** is a multifunctional molecule wherein **I** is a core moiety, **W** stands for one equivalent of hydroxy-reacting group and n₁ is at least 3; **C** is the multifunctional alcohol product mixture from **A** and **B,** wherein L is an ether, ester or urethane link and n₂ is at least 3.

4. The multifunctional alcohol of Claim 3 wherein n₁ and n₂ range from 3 to 6.

5. The multifunctional alcohol of Claim 3 wherein there are at least 2.5 OH groups from A for every equivalent of hydroxy-reacting group, **W,** from **B.**

6. The multifunctional alcohol of Claim 3 wherein L is an ester link.

7. The multifunctional alcohol of Claim 3 having the formula of
**I(̵**L―R₁―Rf―R₂―OH)_{n₂}
wherein n₂ ranges from 3 to 6.

8. The multifunctional alcohol of Claim 3 wherein Rf is a perfluorinated polymethylene moiety having at least 4 carbon atoms.

9. The multifunctional alcohol of Claim 3 wherein Rf is a perfluorinated poly(oxyalkylene) moiety having at least 4 carbon atoms.

10. The multifunctional alcohol of Claim 3 wherein **B** is selected from a group consisting of multifunctional carboxylic acid, acid chloride, ester, and anhydride.

11. The multifunctional alcohol of Claim 3 wherein **B** is selected from 1,3,5-benzenetricarbonyl trichloride, trimethyl-1,3,5-benzenetricarboxylate and 1,2,4-benzenetricarboxylic acid.

12. The multifunctional alcohol of Claim 3 wherein **B** is selected from 1,2,3,4-butanetetracarboxylic acid and tetraethylrimethyl-1,1,2,2-ethanetetracarboxylate.

13. A multifunctional acrylate prepared from the fluorinated multifunctional alcohol of Claim 1.

14. A multifunctional acrylate prepared from the fluorinated multifunctional alcohol of Claim 3.

15. The fluorinated multifunctional acrylate of Claim 13 having a number average molecular weight of at least 500.

16. A multifunctional acrylate prepared from the fluorinated multifunctional alcohol of Claim 7.

17. A polymer coating composition containing at least one acrylate of Claim 13.

18. A multifunctional glycidyl ether prepared from the fluorinated multifunctional alcohol of Claim 1.

19. The multifunctional glycidyl ether of Claim 18 having the formula of 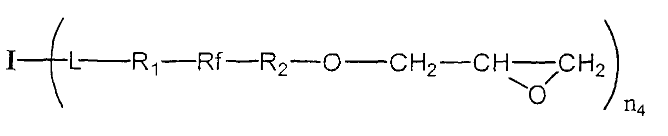 wherein **I** is a multivalent radical; L is selected from a group of ether, ester and urethane links; R₁ and R₂ are monomeric or polymeric divalent radicals such as alkylenediyl, oxyalkylene, alkylene sulfide, arylenediyl, oxyarylene, arylene sulfide, siloxane, and mixtures thereof; Rf is a monomeric or polymeric perfluorinated alkylenediyl, oxyalkylene, arylenediyl, oxyarylene, and mixtures thereof; and n₄ ranges from 3 to 6.

20. A multifunctional vinyl ether prepared from the fluorinated multifunctional alcohol of Claim 1.

21. The multifunctional vinyl ether of Claim 20 having the formula of
**I(̵**L-R₁-Rf-R₂-O-CH=CH₂)_{n₅}
wherein **I** is a multivalent radical; L is selected from a group of ether, ester and urethane links; R₁ and R₂ are monomeric or polymeric divalent radicals such as alkylenediyl, oxyalkylene, alkylene sulfide, arylenediyl, oxyarylene, arylene sulfide, siloxane, and mixtures thereof; Rf is a monomeric or polymeric perfluorinated alkylenediyl, oxyalkylene, arylenediyl, oxyarylene, and mixtures thereof; and n₅ ranges from 3 to 6.

## Patentansprüche

1. Fluorierter polyfunktioneller Alkohol, synthetisiert aus zumindest einem Kernmolekül mit zumindest drei Äquivalenten von hydroxyreagierenden funktionellen Gruppen und zumindest einem fluorierten Molekül mit zumindest zwei Hydroxylgruppen.

2. Polyfunktioneller Alkohol nach Anspruch 1, wobei zumindest 1,5 Äquivalente von Hydroxylgruppen von dem fluorierten Molekül für jede hydroxyreagierende Gruppe von dem Kernmolekül vorhanden sind.

3. Polyfunktioneller Alkohol nach Anspruch 1, synthetisiert unter Einsatz des Reaktionsschemas 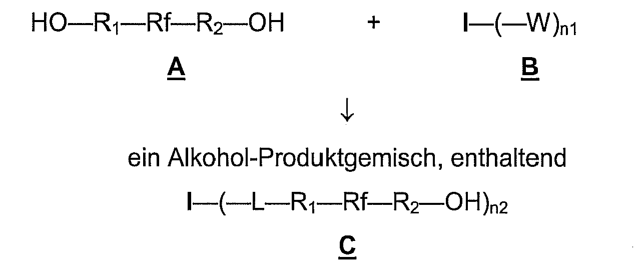 wobei **A** ein fluoriertes Monomer oder Polymer mit zwei Hydroxylgruppen ist, wobei Rf ein monomeres oder polymeres perfluoriertes Alkylendiyl, Oxyalkylen, Arylendiyl, Oxyarylen und Gemische davon ist und R₁ und R₂ monomere oder polymere zweiwertige Komponenten sind, wie Alkylendiyl, Oxyalkylen, Alkylensulfid, Arylendiyl, Oxyarylen, Arylensulfid, Siloxan und Gemische davon; **B** ein polyfunktionelles Molekül ist, wobei **I** eine Kernkomponente ist, **W** für ein Äquivalent einer hydroxyreagierenden Gruppe steht und n₁ zumindest 3 ist; **C** das polyfunktionelle Alkohol-Produktgemisch aus **A** und **B** ist, wobei L ein Ether-, Ester- oder Urethan-Bindeglied ist und n₂ zumindest 3 ist.

4. Polyfunktioneller Alkohol nach Anspruch 3, wobei n₁ und n₂ im Bereich von 3 bis 6 liegen.

5. Polyfunktioneller Alkohol nach Anspruch 3, wobei zumindest 2,5 OH-Gruppen von **A** für jedes Äquivalent einer hydroxyreagierenden Gruppe **W** von **B** vorhanden sind.

6. Polyfunktioneller Alkohol nach Anspruch 3, wobei L ein Ester-Bindeglied ist.

7. Polyfunktioneller Alkohol nach Anspruch 3 mit der Formel
I―(―L―R₁―Rf―R₂―OH)ₙ₂
wobei n₂ im Bereich von 3 bis 6 liegt.

8. Polyfunktioneller Alkohol nach Anspruch 3, wobei Rf eine perfluorierte Polymethylen-Komponente mit zumindest 4 Kohlenstoffatomen ist.

9. Polyfunktioneller Alkohol nach Anspruch 3, wobei Rf eine perfluorierte Poly(oxyalkylen)-Komponente mit zumindest 4 Kohlenstoffatomen ist.

10. Polyfunktioneller Alkohol nach Anspruch 3, wobei **B** ausgewählt ist aus einer Gruppe, die aus polyfunktioneller Carbonsäure, Säurechlorid, -ester und - anhydrid besteht.

11. Polyfunktioneller Alkohol nach Anspruch 3, wobei **B** ausgewählt ist aus 1,3,5-Benzentricarbonyltrichlorid, Trimethyl-1,3,5-Benzentricarboxylat und 1,2,4-Benzentricarbonsäure.

12. Polyfunktioneller Alkohol nach Anspruch 3, wobei **B** ausgewählt ist aus 1,2,3,4-Butantetracarbonsäure und Tetraethyltrimethyl-1,1,2,2-ethantetracarboxylat.

13. Polyfunktionelles Acrylat, hergestellt aus dem fluorierten polyfunktionellen Alkohol nach Anspruch 1.

14. Polyfunktionelles Acrylat, hergestellt aus dem fluorierten polyfunktionellen Alkohol nach Anspruch 3.

15. Fluoriertes polyfunktionelles Acrylat nach Anspruch 13 mit einem Zahlenmittel-Molekulargewicht von zumindest 500.

16. Polyfunktionelles Acrylat, hergestellt aus dem fluorierten polyfunktionellen Alkohol nach Anspruch 7.

17. Polymer-Beschichtungszusammensetzung, die zumindest ein Acrylat nach Anspruch 13 enthält.

18. Polyfunktioneller Glycidylether, hergestellt aus dem fluorierten polyfunktionellen Alkohol nach Anspruch 1.

19. Polyfunktioneller Glycidylether nach Anspruch 18 mit der Formel 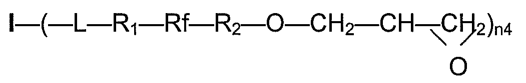 wobei **I** ein mehrwertiger Rest ist; L ausgewählt ist aus einer Gruppe aus Ether-, Ester- und Urethan-Bindegliedern; R₁ und R₂ monomere oder polymere zweiwertige Reste sind, wie Alkylendiyl, Oxyalkylen, Alkylensulfid, Arylendiyl, Oxyarylen, Arylensulfid, Siloxan und Gemische davon; Rf ein monomeres oder polymeres perfluoriertes Alkylendiyl, Oxyalkylen, Arylendiyl, Oxyarylen und Gemische davon ist; und n₄ im Bereich von 3 bis 6 liegt.

20. Polyfunktioneller Vinylether, hergestellt aus dem fluorierten polyfunktionellen Alkohol nach Anspruch 1.

21. Polyfunktioneller Vinylether nach Anspruch 20 mit der Formel
**I**―(―L―R₁―Rf―R₂―O―CH==CH₂)ₙ₅
wobei **I** ein mehrwertiger Rest ist; L ausgewählt ist aus einer Gruppe aus Ether-, Ester- und Urethan-Bindegliedern; R₁ und R₂ monomere oder polymere zweiwertige Reste sind, wie Alkylendiyl, Oxyalkylen, Alkylensulfid, Arylendiyl, Oxyarylen, Arylensulfid, Siloxan und Gemische davon; Rf ein monomeres oder polymeres perfluoriertes Alkylendiyl, Oxyalkylen, Arylendiyl, Oxyarylen und Gemische davon ist; und n₅ im Bereich von 3 bis 6 liegt.

## Revendications

1. Alcool multifonctionnel fluoré synthétisé à partir d'au moins une molécule de coeur ayant au moins trois équivalents de groupes fonctionnels réagissant avec hydroxy et d'au moins une molécule fluorée ayant au moins deux groupes hydroxyle.

2. Alcool multifonctionnel selon la revendication 1 où il y a au moins 1,5 équivalent de groupes hydroxyle provenant de la molécule fluorée pour chaque groupe réagissant avec hydroxy provenant de la molécule de coeur.

3. Alcool multifonctionnel selon la revendication 1 synthétisé au moyen du schéma réactionnel : 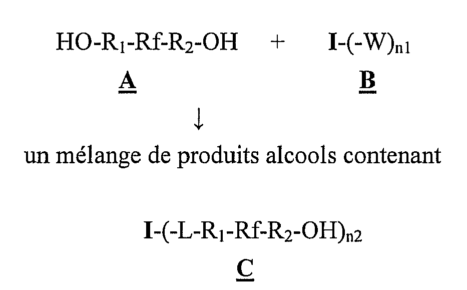 où A est un monomère ou polymère fluoré ayant deux groupes hydroxyle, où Rf est un alkylènediyle, oxyalkylène, arylènediyle, oxyarylène perfluoré monomère ou polymère, et leurs mélanges, et R₁ et R₂ sont des groupements divalents monomères ou polymères comme alkylènediyle, oxyalkylène, sulfure d'alkylène, arylènediyle, oxyarylène, sulfure d'arylène, siloxane, et leurs mélanges ; **B** est une molécule multifonctionnelle où **I** est un groupement de coeur, **W** représente un équivalent de groupe réagissant avec hydroxy et n₁ est au moins 3 ; **C** est le mélange de produits alcools multifonctionnels provenant de A et B, où L est une liaison éther, ester ou uréthane et n₂ est au moins 3.

4. Alcool multifonctionnel selon la revendication 3 où n₁ et n₂ vont de 3 à 6.

5. Alcool multifonctionnel selon la revendication 3 où il y a au moins 2,5 groupes OH provenant de **A** pour chaque équivalent de groupe réagissant avec hydroxy, **W**, provenant de **B**.

6. Alcool multifonctionnel selon la revendication 3 ou L est une liaison ester.

7. Alcool multifonctionnel selon la revendication 3 ayant la formule de
I-(-L-R₁-Rf-R₂-OH)ₙ₂
où n₂ va de 3 à 6.

8. Alcool multifonctionnel selon la revendication 3 ou Rf est un groupement polyméthylène perfluoré ayant au moins 4 atomes de carbone.

9. Alcool multifonctionnel selon la revendication 3 ou Rf est un groupement poly(oxyalkylène) perfluoré ayant au moins 4 atomes de carbone.

10. Alcool multifonctionnel selon la revendication 3 où **B** est choisi dans un groupe consistant en un acide carboxylique, chlorure d'acide, ester et anhydride multifonctionnel.

11. Alcool multifonctionnel selon la revendication 3 où **B** est choisi parmi le trichlorure de 1,3,5-benzènetricarbonyle, le 1,3,5-benzènetricarboxylate de triméthyle et l'acide 1,2,4-benzènetricarboxylique.

12. Alcool multifonctionnel selon la revendication 3 où **B** est choisi parmi l'acide 1,2,3,4-butanetétracarboxylique est le triméthyl-1,1,2,2-éthanetétracarboxylate de tétraéthyle.

13. Acrylate multifonctionnel préparé à partir de l'alcool multifonctionnel fluoré selon la revendication 1.

14. Acrylate multifonctionnel préparé à partir de l'alcool multifonctionnel fluoré selon la revendication 3.

15. Acrylate multifonctionnel fluoré selon la revendication 13 ayant une masse moléculaire moyenne en nombre d'au moins 500.

16. Acrylate multifonctionnel préparé à partir de l'alcool multifonctionnel fluoré selon la revendication 7.

17. Composition de revêtement polymère contenant au moins un acrylate selon la revendication 13.

18. Glycidyléther multifonctionnel préparé à partir de l'alcool multifonctionnel fluoré selon la revendication 1.

19. Glycidyléther multifonctionnel selon la revendication 18 ayant la formule de 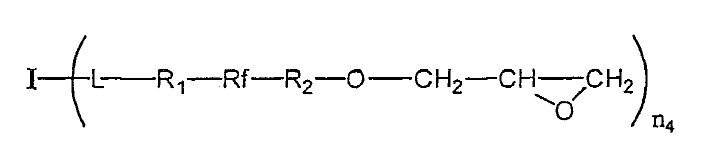 où **I** est un radical multivalent ; L est choisi dans un groupe de liaisons éther, ester et uréthane ; R₁ et R₂ sont des radicaux divalents monomères ou polymères comme alkylènediyle, oxyalkylène, sulfure d'alkylène, arylènediyle, oxyarylène, sulfure d'arylène, siloxane, et leurs mélanges ; Rf est un alkylènediyle, oxyalkylène, arylènediyle, oxyarylène perfluoré monomère ou polymère, et leurs mélanges ; est n₄ va de 3 à 6.

20. Vinyléther multifonctionnel préparé à partir de l'alcool multifonctionnel fluoré selon la revendication 1.

21. Vinyléther multifonctionnel selon la revendication 20 ayant la formule de
I-(-L-R₁-Rf-R₂-O-CH=CH₂)ₙ₅
où **I** est un radical multivalent ; L est choisi dans un groupe de liaisons éther, ester et uréthane ; R₁ et R₂ sont des radicaux divalents monomères ou polymères comme alkylènediyle, oxyalkylène, sulfure d'alkylène, arylènediyle, oxyarylène, sulfure d'arylène, siloxane, et leurs mélanges ; Rf est un allcylènediyle, oxyalkylène, arylènediyle, oxyarylène perfluoré monomère ou polymère, et leurs mélanges ; est n₅ va de 3 à 6.
